# EUROPEAN PATENT APPLICATION

(11) **EP 2 116 139 A1**
(43) Date of publication of application: **11.11.2009**
(21) Application number: 08155853.8
(22) Date of filing: 08.05.2008
(51) Int. Cl.: A23L 1/29, A61K 31/70, A61P 25/00, A61P 1/00

(54) **Sialic acid to support brain health in the elderly**

(71) Applicant: Nestec S.A., 1800 Vevey (CH)
(72) Inventor: Sprenger, Norbert, 1073, Savigny (CH); Vidal, Karine, 1010, Lausanne (CH); Cherbut, Christine, 1009, Pully (CH); Jann, Alfred, 74500, Publier (FR)
(74) Representative: Marquardt, Ulf

(57) **Abstract**

The present invention generally relates to the field of brain and/or central and/or peripheral nervous system health, in particular to maintaining and improving brain health in the elderly. One embodiment of the present invention relates to the use of a food product enriched with sialic acid for the preparation of a composition to maintain and re-establish central and peripheral nervous system health in the elderly.

## Description

The present invention generally relates to the field of brain and/or central and/or peripheral nervous system health, in particular to maintaining and improving brain health in the elderly. One embodiment of the present invention relates to the use of a food product enriched with sialic acid for the preparation of a composition to maintain and re-establish central and peripheral nervous system health in the elderly.

Many persons live to old age with a well-functioning brain. But many functional and structural changes occur in the nervous system during aging, and not every person's nervous system can cope with the changes to maintain a fully functional nervous system. Reasons for the inter-individual differences are manifold and include genotype, nutrition and behaviour.

Aging is associated with functional alterations of neurons and a progressive neuronal loss of the central and peripheral nervous system. For example, age related neuronal losses in the enteric nervous system affect primarily cholinergic neurons. To a certain extent and within the limits of each individual's respective lifestyle such neuronal losses can be functionally compensated for by plasticity of the nervous system. This includes functional adaptation by remaining neurons and/or neurogenesis.

Functional deficits in aged persons related to neuronal loss and functional alterations of neurons include (i) in the central nervous system loss of long term memory, short term memory, learning ability, and/or motor balance and coordination, and (ii) in the peripheral nervous system constipation, incontinence, evacuation disorders, gastrointestinal reflux and dysphagia. Another condition frequently associated with age is dry-mouth-feeling linked to deficits in salivation and increased intestinal permeability, indicating a reduced gut barrier function and generally linked to malnutrition in the elderly.

Traditionally there has been very little research in geriatric mental health despite the fact that some seniors suffer from a wide array of mental illnesses and are often prescribed very potent drugs.

There remains to be a need in the art for a composition that does not have the disadvantages of the medications used so far.

The present inventors have addressed this need.

Consequently, it was the object of the present invention to provide the art with a composition that is available to everybody that can be administered without the risk of unwanted side effects that is inexpensive and that can be used to maintain and re-establish central and peripheral nervous system health in elderly people.

The present inventors were surprised to see that they could achieve this object by a use in accordance with claim 1.

Sialic acids (SiAc) are a family of charged nine carbon monosaccharides derived from neuraminic acid (NeuAc). NeuAc is the only sialic acid normally formed in humans. In other vertebrates, for example N-glycolylneuraminic acids (NeuGc), are also present.

Today, sialic acids are frequently used in the field of infant nutrition. For example, a possible involvement of SiAc in the cognitive development of infants was summarized by Wang (Wang, B. and Brand-Miller, J. (2003) Eur.J.Clin.Nutr. Nov;57(11):1351-69). Briefly, studies comparing breast-fed and formula-fed infants demonstrate that a higher NeuAc content of breast milk compared to a normal infant formula correlates with an increased NeuAc content of infants saliva and brain. However, behavioural effects of NeuAc supplementation in humans are not available. Nevertheless it is speculated that supplementation of cows milk with NeuAc would provide the cows milk with human milk attributes, which might have an impact on brain development of children.

Natural sources rich in SiAc, for example NeuAc, are, e.g., human milk, elephant milk, Indian buffalo milk, meat, eggs and fish.

The present inventors have now found, that sialic acid can not only be successfully administered to infants, but is especially effective in maintaining and re-establishing central and peripheral nervous system health in the elderly, for example by improving cholinergic neuronal function, general activity and gut health.

The administration of the composition of the present invention to aged animals was found to lead to an elevated sialylation in the brain. This is seen for example by an increased sialylation of ganglioside (sialyl-lactosylceramides) enriched brain preparations. Sialylation and especially gangliosides are important factors stabilizing neuronal integrity and allowing for neuronal plasticity equally in the central and peripheral nervous system.

Without wishing to be bound by theory, the present inventors assume that the observed effect can be explained as follows:

Sialic acid is a building block terminally displayed on numerous compounds - glycolipids and glycoproteins - involved in cell-cell communication, cell adhesion, cell migration and cell sensing. Gangliosides are glycolipids carrying one or more sialic acids. Brain aging might be associated with a loss of gangliosides. At the same time, the endogenous synthetic capacity to form sialic acid is reduced with age.

The present inventors provide sialic acid with the food to elderly subjects, boosting endogenous gangliosides, for example by a sialic acid enriched diet. To the inventors' surprise, sialic acid feeding of aged mice increased ganglioside bound sialic acid levels in the brain of aged rats. This indicates that nervous system gangliosides can be altered through nutrition in aged animals.

The inventors further investigated the physiological consequence of the increased ganglioside levels. The wood gnawing behaviour of rats was evaluated to assess if aged animals show a different activity as compared to young animals and whether SiAc feeding affects this behaviour.

In general aged animals showed less wood gnawing activity.

Surprisingly, however, sialic acid feeding of aged rats resulted in a tendency to show a higher general activity as measured by the wood gnawing activity. This indicates that brain function and brain health was positively affected by the nutritional intervention with a sialic acid enriched diet in aged animals.

As cholinergic neurons are primarily affected by age, the inventors explored the function of cholinergic neurons. This was done by injecting a cholinergic agonist and by evaluating as a functional read-out the resulting salivation over time.

Aged animals showed less salivation as compared to young animals. Surprisingly, sialic acid feeding lead to an increased stimulated salivation equal to the salivation found in young animals. This allows the conclusion that the nutritional intervention with a sialic acid enriched diet ameliorated cholinergic nervous system function and, consequently, nervous system health.

Consequently, one embodiment of the present invention is a composition comprising sialic acid to maintain and re-establish central and peripheral nervous system health in the elderly.

A further embodiment of the present invention is a composition comprising sialic acid to maintain and re-establish brain health.

The present invention also relates to the use of sialic acid containing ingredient for the preparation of a composition to maintain and re-establish central and peripheral nervous system health in the elderly, and/or to maintain and/or re-establish brain health.

For this purpose it is preferred if the ingredient and/or the composition is enriched with sialic acid.

A subject is considered as "elderly" if it has surpassed the first half of its average expected lifespan in its country of origin, preferably, if it has surpassed the first two thirds of the average expected lifespan in its country of origin, more preferably if it has surpassed the first three quarters of the average expected lifespan in its country of origin, most preferred if it has surpassed the first four fifths of the average expected lifespan in its country of origin.

The composition may be administered to humans or animals, in particular pets, companion animals and/or livestock.

The present invention relates also to a composition enriched with SiAc. Any sialic acid may be used for the purposes of the present invention. However, it is preferred if the sialic acid has the following formula 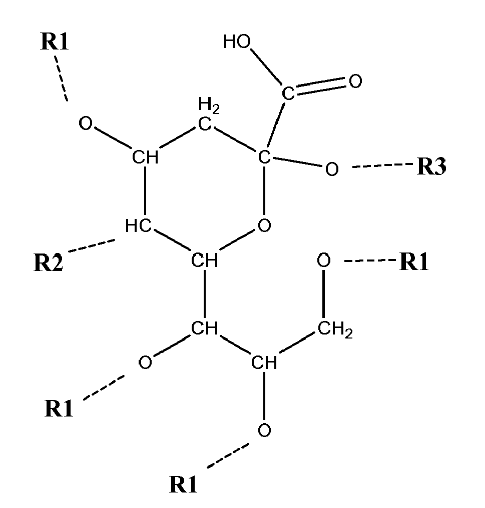
**R1** = H, acetyl, lactyl, methyl, sulfate, phosphate, anhydro, sialic acid, fucose, glucose, or galactose
**R2** = N-acetyl, N-glycolyl, amino, hydroxyl, N-glycolyl-O-acetyl, or N-glycolyl-O-methyl
**R3** = H, galactose, N-acetylglucosamine, N-acetylgalactosamine, sialic acid, or N-glycolylneuraminic acid

R1 may be selected from the group consisting of H, acetyl, lactyl, methyl, sulfate, phosphate, anhydrosialic acid, fucose, glucose and/or galactose.

R2 may be selected from the group consisting of N-acetyl, N-glycolyl, amino, hydroxyl, N-glycolyll-O-acetyl, and/or N-glycolyl-O-methyl.

R3 may be selected from the group consisting of H, galactose, N-acetylglucosime, N-acetylgalactosamine, sialic acid, and/or n-glycolylneuraminic acid.

The groups in position R1 may be identical or different from each other.

In a particularly preferred embodiment of the present invention, the sialic acid is N-acetylneuraminic acid with R1=H, R2=N-acetyl and R3=H. According to a further preferred embodiment of the present invention the sialic acid may be selected from the group consisting of 2-keto-5-acetamido-3,5-dideoxy-d-glycero-d-galactononulosonic acid (Neu5Ac) and 2-keto-3-deoxy-d-glycero-d-galactonononic acid (KDN) or mixtures thereof.

Sialic acid as used in the present invention comprises N-Acetylneuraminic acid, which has the following synonyms and abbreviations: o-Sialic acid; 5-Acetamido-3,5-dideoxy-D-glycero-D-galacto-2-nonulosonic acid; 5-Acetamido-3,5-dideoxy-D-glycero-D-galactonulosonic acid; Aceneuramic acid; N-acetyl-neuraminate; N-Acetylneuraminic acid; NANA, and Neu5Ac.

The composition of the present invention may be a nutritional composition, a nutraceutical, a drink, a food additive or a medicament. A food additive or a medicament may be in the form of tablets, capsules, pastilles or a liquid for example. Food additives or medicaments are preferably provided as sustained release formulations, allowing a constant SiAc supply for prolonged times.

The composition is preferably selected from the group consisting of milk powder based products; instant drinks; ready-to-drink formulations; nutritional powders; nutritional liquids; milk-based products, in particular yoghurts or ice cream; cereal products; beverages; water; coffee; cappuccino; malt drinks; chocolate flavoured drinks; culinary products; soups; topical creams; suppositories; tablets; syrups; and formulations for transdermal applications.

Milk may be any milk obtainable from animal or plant sources and is preferably cows milk, human milk, sheep milk, goat milk, horse milk, camel milk, rice milk or soy milk.

Instead of milk, also milk derived protein fractions or colostrum may be used.

The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. It may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. Further, it may contain an organic or inorganic carrier material suitable for oral or enteral administration as well as vitamins, minerals trace elements and other micronutrients in accordance with the recommendations of Government bodies such as the USRDA. For example, it may contain one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 g iodine, 5 to 15 g selenium, 1000 to 3000 g beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 g Vitamin B12, 100 to 800 g folic acid, 30 to 70 g biotin, 1 to 5 g Vitamin D, 3 to 10 IU Vitamin E.

The composition of the present invention may contain a protein source, a carbohydrate source and/or a lipid source.

Any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. Very positive results for the purpose of the present invention were achieved when the protein fraction comprised threonine in an amount of between about 8 and 22% of the total number of amino acids of the protein fraction.

If the composition includes a fat source, the fat source more preferably provides 5% to 40% of the energy of the formula; for example 20% to 30% of the energy. DHA may be added. A suitable fat profile may be obtained using a blend of canola oil, corn oil and high-oleic acid sunflower oil.

A source of carbohydrates may more preferably provide between 40% to 80% of the energy of the composition. Any suitable carbohydrate may be used, for example sucrose, lactose, glucose, fructose, corn syrup solids, maltodextrins, and mixtures thereof.

According to a particular preferred embodiment of the present invention, the protein fraction comprises N-acetylneuraminic acid (NeuAc) and is characterized by a threonine rich peptide/protein backbone (between 8 and 22 % of total number of amino acids) and a NeuAc content of 7 to 25 % by mass.

The composition of the present invention may be used to elevate sialylation in the central and peripheral nervous system, and/or to stabilize neuronal integrity.

It may also be used to support neuronal repair and/or plasticity, in particular in the central and/or in the peripheral nervous system.

Further, the composition of the present invention may be used to improve signal conduction velocity, in particular in the central and/or in the peripheral nervous system.

Even further, the composition of the present invention may be used to support post synaptic responses, in particular in the central and/or in the peripheral nervous system.

Finally, the composition of the present invention may be used to improve long term memory, short term memory, learning, and/or motor balance and coordination.

Typical conditions that may be treated or prevented by the use of the composition of the present invention include for example age related brain disorders, degenerative brain disorders, mood, anxiety and psychotic disorders as well as the emotional, behavioural and cognitive complications of brain diseases such as Alzheimer's and Parkinson's, and the effects of stroke. Further examples are senile dementia, forgetfulness, cognitive impairment.

Typical conditions related to impaired neuronal function in the gut that may be treated or prevented by the use of the composition of the present invention include for example age related gastrointestinal barrier disfunction, constipation, incontinence, evacuation disorders, dysphagia and gastrointestinal reflux.

The effects of the composition may further be enhanced by adding to the composition at least one neuron protective agent, such as antioxidant plant extracts, vitamins and/or micronutrients.

The composition may also comprise a probiotic micro-organism and/or a prebiotic such as fructooligosaccharides, galactosyloligosaccharides, pectins and/or hydrolysates thereof.

Prebiotics are in particular preferred if the composition comprises probiotics, since the presence of probiotics and prebiotics produces a synergistic effect.

"Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

All probiotic micro-organisms may be used in accordance with the present invention. Preferably, they are selected from the group consisting of Bifidobacterium, Lactobacillus, Streptococcus and Saccharomyces or mixtures thereof, in particular selected from the group consisting of *Bifidobacterium longum, Bifidobacterium lactis, Lactobacillus acidophilus, Lactobacillus rhamnosus, Lactobacillus paracasei, Lactobacillus johnsonii, Lactobacillus plantarum, Lactobacillus salivarius, Enterococcus faecium, Saccharomyces boulardii* and *Lactobacillus reuteri* or mixtures thereof, preferably selected from the group consisting of *Lactobacillus johnsonii* (NCC533; CNCM 1-1225), *Bifidobacterium longum* (NCC490; CNCM I-2170), *Bifidobacterium longum* (NCC2705; CNCM I-2618), *Bifidobacterium lactis* (2818; CNCM 1-3446), *Lactobacillus paracasei* (NCC2461; CNCM I-2116), *Lactobacillus rhamnosus* GG (ATCC53103), *Lactobacillus rhamnosus* (NCC4007; CGMCC 1.3724), *Enterococcus faecium* SF 68 (NCIMB10415), and mixtures thereof.

"Prebiotic" means food substances that promote the growth of probiotics in the intestines. They are not broken down in the stomach and/or upper intestine or absorbed in the GI tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

The prebiotics that may be used in accordance with the present inventions are not particularly limited and include all food substances that promote the growth of probiotics in the intestines. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (IOS), isomalto-oligosaccharides, xylo-oligosaccharides, oligosaccharides of soy, glycosylsucrose (GS), lactosucrose (LS), lactulose (LA), palatinose-oligosaccharides (PAO), malto-oligosaccharides (MOS), gums and/or hydrolysates thereof, pectins and/or hydrolysates thereof.

The effect of sialic acid in the composition of the present invention was found to be essentially dose dependent. Small amounts will produce smaller effects and large amounts may be to large so that the body cannot utilize all SiAc provided. The exact amount of SiAc to be provided will depend on the subject to be treated and on its condition, for example.

While in generally every amount of SiAc will produce a beneficial effect it was found to be in particularly preferred if the sialic acid is present in the composition in an amount of 1 mg-250 mg/g dry mass of the composition.

The sialic acid may be administered in a daily amount of 1mg-2g /kg body weight, preferably 0,025 g to 0,8 g/kg body weight of the subject to be treated.

Those skilled in the art will understand that they can freely combine all features of the present invention described herein, without departing from the scope of the invention as disclosed. In particular, features described for the uses of the present invention may be applied to the composition of the present invention and vice versa.

Further advantages and features of the present invention are apparent from the following Examples and Figures.

### Figures :

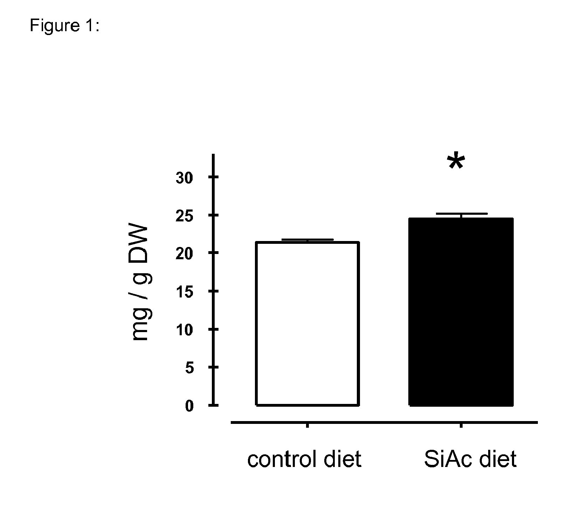

Figure 1 shows that the sialic acid feeding of aged mice leads to increased ganglioside bound sialic acid levels in the right brainhalf (A),. Aged mice (18 months) were fed for 41 days with a semisynthetic diet containing either <0.1 g/100g sialic acid (control diet) or 0.8 g/100g sialic acid (Sia diet) and their brain sialic acid levels were compared. Mean values and standard error of the mean are presented. N=10; * indicates significant difference at a p=0.0025 by t-test.

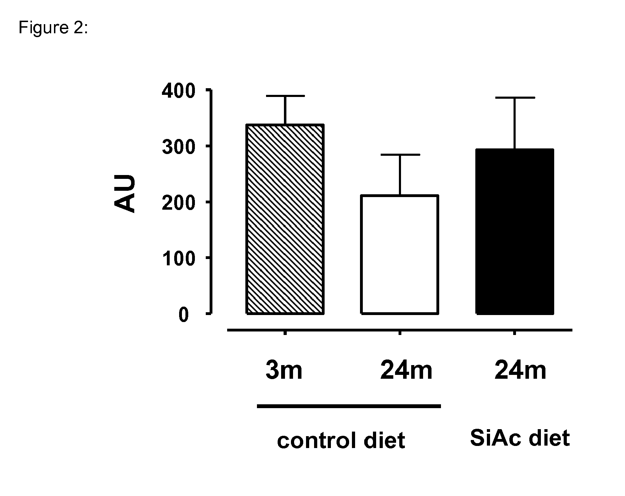

Figure 2 demonstrates that aged rats show a tendency to be less active as measured by wood gnawing when compared to young adult rats, but show a tendency to increase activity upon feeding a sialic acid rich diet. Mean values and standard error of the mean of the area under the curve of the cumulative wood gnawing are presented. N=9-10

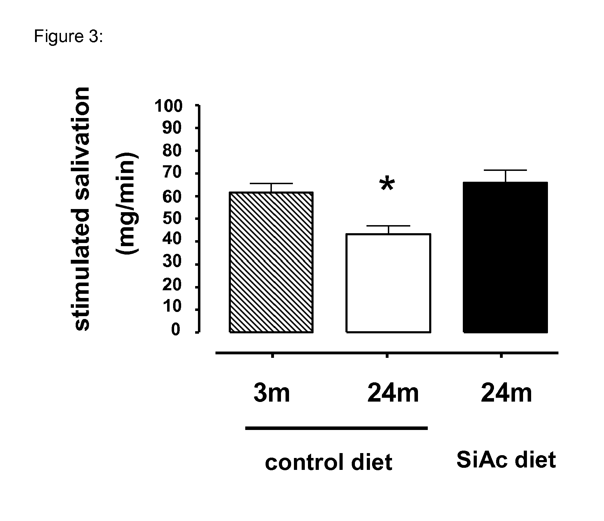

Figure 3 demonstrates that aged rats show less cholinergic neuronal activity as measured by stimulated saliva production per time when compared to young adult rats, but show a significantly increased neuronal activity upon feeding a sialic acid rich diet. Mean values and standard error of the mean are presented. N=9-10; * indicates significant difference at a p=0.0035 comparing 3 and 24 months on control diet and at a p=0.0024 comparing 24 months control and Sia diet by t-test.

### Examples:

### Example 1:

Aged mice (18 months) were fed a semisynthetic diet containing sialic acid at <0.1 g/100g diet (control diet) and a semisynthetic diet containing sialic acid at 0.8 g/100g diet (Sia diet). After 41 days mice were anaesthesized and euthanized by exhaustive bleeding. Brains were removed, halfed into a right and left lobe and frozen immediately in liquid nitrogen. Brain ganglioside and protein bound sialic acid levels were determined after lipid extraction and ganglioside enrichment. Briefly, brains were homogenized in ice-cold water and lipids were extracted with 5.4 volumes of methanol:chloroform (2:1). The resulting pellet was resuspended with water lipids were re-extracted with 4 volumes methanol:chloroform (2:1). The combined supernatants were subjected with 0.25 volumes water. The resulting upper aqueous phase was collected and the lower organic phase re-extracted with 0.2 volumes methanol and 0.15 volumes 10 mM KCI in water. After gently mixing the resulting upper phase was combined with the first aqueous extract and dried in a stream of nitrogen. The thus enriched ganglioside fraction did not contain measurable protein and was used to determine ganglioside bound sialic acid. Sialic acid levels (NeuAc) were determined after mild acid hydrolysis with 2 M acetic acid at 80°C for 3 hr by HPAEC-PAD analysis on an ICS3000 (Dionex) machine equipped with a pulsed amperometric detector and a CarboPac PA1 analytical column. Authentic N-acetylneuraminic acid were used as external standards for quantification.

### Example 2:

Young adult (3 months) and aged rats (24 months) were fed a semisynthetic diet containing sialic acid at a concentration of 0.15 g/100g diet (control) or a semisynthetic diet additionally supplemented with sialic acid to a final concentration of 0.9 g/100g diet (Sia).
During the 3 weeks intervention each rat caged individually got a piece of wood, which was weighed every 3 days and replaced with a new piece if needed. This allowed calculating a cumulative wood gnawing activity, which is a rough estimator for the general activity of each animal.

As seen in Figure 2, the aged animals were slightly less active as compared to the young animals. Upon sialic acid feeding a tendency to increased activity was found similar to the one found with young animals. This indicates that sialic acid feeding affected behaviour of aged animals.

After 3 weeks on the experimental diet neuronal activity of cholinergic neurons was assessed. To this end rats were injected pilocarpine (IP, 1.5 mg/kg body weight for 24 months rats and 2 mg/kg body weight for 3 months rats). Pilocarpine is a muscarinic agonist acting on cholinergic neurons, which leads to salivation. Thus stimulated saliva collection was timed with a chronometer. After about 7 minutes collection was stopped.

As seen in Figure 3, aged animals showed less stimulated salivation as compared to young animals. Upon sialic acid feeding the aged animals reached similar stimulated salivation values as the young animals. This indicates that sialic acid feeding helped functional recovery of cholinergic neuron function that decreased with age.

The observations made here show that nervous system health was re-established in aged animals by providing sialic acid via a nutritional approach.

## Claims

1. Use of sialic acid containing ingredient for the preparation of a composition to maintain and re-establish central and peripheral nervous system health in the elderly.

2. Use in accordance with claim 1 to maintain and/or re-establish brain health.

3. Use in accordance with one of the preceding claims wherein the sialic acid is selected from the group consisting of compounds with the following formula 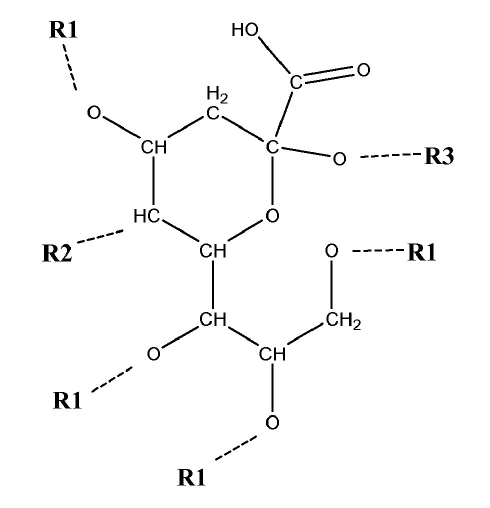
**R1** = H, acetyl, lactyl, methyl, sulfate, phosphate, anhydro, sialic acid, fucose, glucose, or galactose
**R2** = N-acetyl, N-glycolyl, amino, hydroxyl, N-glycolyl-O-acetyl, or N-glycolyl-O-methyl
**R3** = H, galactose, N-acetylglucosamine, N-acetylgalactosamine, sialic acid, or N-glycolylneuraminic acid
or mixtures thereof, and preferably the sialic acid is selected from the group consisting of 2-keto-5-acetamido-3,5-dideoxy-d-glycero-d-galactononulosonic acid (Neu5Ac) and 2-keto-3-deoxy-d-glycero-d-galactonononic acid (KDN) or mixtures thereof.

4. Use in accordance with one of the preceding claims to elevate sialylation in the central and peripheral nervous system, and/or to stabilize neuronal integrity.

5. Use in accordance with one of the preceding claims to support neuronal repair and/or plasticity, in particular in the central and/or in the peripheral nervous system.

6. Use in accordance with one of the preceding claims to improve signal conduction velocity, in particular in the central and/or in the peripheral nervous system.

7. Use in accordance with one of the preceding claims to support post synaptic responses, in particular in the central and/or in the peripheral nervous system.

8. Use in accordance with one of the preceding claims to improve long term memory, short term memory, learning, and/or motor balance and coordination.

9. Use in accordance with one of the preceding claims to improve general activity.

10. Use in accordance with one of the preceding claims wherein the composition is a pharmaceutical composition, a food product, a food additive or a nutraceutical.

11. Use in accordance with one of the preceding claims wherein the composition further comprises a neuron protective agent, such as antioxidant plant extracts, vitamins or micronutrients; and/or a probiotic micro-organism and/or a prebiotic, preferably fructooligosaccharides, galactosyloligosaccharides, pectins and/or hydrolysates thereof.

12. Use in accordance with one of the preceding claims wherein the sialic acid is present in the composition in an amount of 1mg-250 mg/g dry mass of the composition.

13. Use in accordance with one of the preceding claims wherein the sialic acid is to be administered in a daily amount of 1 mg-2g /kg body weight to the subject.

14. Use in accordance with one of the preceding claims wherein the composition is selected from the group consisting of milk powder based products; instant drinks; ready-to-drink formulations; nutritional powders; nutritional liquids, milk-based products, in particular yoghurts or ice cream; cereal products; biscuits; cereal bars; beverages; water; coffee; cappuccino; tea; fruit juices; malt drinks; chocolate flavoured drinks; culinary products; soups; confectionary products; chocolates; topical creams, suppositories, tablets, syrups, and formulations for transdermal applications.
